# EUROPÄISCHE PATENTSCHRIFT

(11) **EP 1 474 183 B1**
(45) Veröffentlichungstag und Bekanntmachung des Hinweises auf die Patenterteilung: **29.11.2006**
(21) Anmeldenummer: 03706436.7
(22) Anmeldetag: 06.02.2003
(51) Int. Cl.: A61L 15/40, D06M 13/224

(54) **WÄSSERIGE MITTEL ZUR HAUTFREUNDLICHEN AUSRÜSTUNG VON VLIESSTOFFEN**
AQUEOUS MEDIUM FOR PRODUCTION OF SKIN FRIENDLY NON-WOVEN MATERIALS
AGENTS AQUEUX POUR MUNIR DES NON-TISSES D'UN APPRET AGREABLE POUR LA PEAU

(30) Priorität: 15.02.2002 DE 10206617
(43) Veröffentlichungstag der Anmeldung: 10.11.2004
(73) Patentinhaber: Cognis IP Management GmbH, 40589 Düsseldorf (DE)
(72) Erfinder: MATHIS, Raymond, 40627 Düsseldorf (DE); NEUSS, Michael, 50997 Köln (DE); WILD, Christine, 40724 Hilden (DE)
(86) Internationale Anmeldenummer: PCT/EP2003/001155
(87) Internationale Veröffentlichungsnummer: WO 2003/068282

(56) Entgegenhaltungen:
- EP-A- 0 103 854
- WO-A-00/04230
- WO-A-98/32413
- DE-C- 3 309 530
- DE-C- 19 547 986

## Beschreibung

Die vorliegende Erfindung betrifft wässerige Mittel zur Ausrüstung von Vliesstoffen, insbesondere von Vliesstoffen, die in Hygieneprodukten eingesetzt werden können.

Bei der Herstellung von Hygieneartikeln, wie Windeln oder Damenbinden, werden absorbierende Materialien verwendet, um wässrige Flüssigkeiten aufzunehmen. Um den direkten Kontakt mit dem absorbierenden Material beim Tragen zu verhindern und den Tragekomfort zu erhöhen wird dieses Material mit einem dünnen, wasserdurchlässigen Vliesstoff umhüllt. Derartige Vliesstoffe werden üblicherweise aus synthetischen Fasern, wie Polyolefin- oder Polyesterfasern hergestellt, da diese Fasern preiswert zu produzieren sind, gute mechanische Eigenschaften aufweisen und thermisch belastbar sind.

Derartige Vliesstoffe werden im Bereich der Hygieneartikel zunehmend mit hautfreundlichen Lotionen ausgestattet, um die Verträglichkeit und den Tragekomfort allgemein zu verbessern. Beispielsweise wird in der DE 33 09 530 C1 eine hygienische Absorptionsvorlage beschrieben, welche mit einer Hautpflegemasse imprägniert ist, die aus Triglyceriden und/oder Partialglyceriden der Kokosölfettsäuren mit 8 bis 18 C-Atomen bestehen. Damit diese Präparationen auch während des Tragens problemlos vom Vliesstoff auf die Haut übergehen können, sind die Triglyceridmischungen der DE 33 09 530 so ausgewählt, dass sie einen Steigschmelzpunkt im Bereich von 35 bis 40 °C aufweisen.

Ein anderer Ansatz, hautpflegende Substanzen während des Tragens von Hygieneartikeln auf die Haut zu übertragen, findet sich in der WO 96/16682. Beschrieben wird dort eine Windel, deren inneres Abdeckvlies mit einer bei 20 °C festen oder halbfesten Lotion präpariert ist, die beim Tragen auf die Haut des Trägers übergeht. Diese Lotionen enthalten von 10 bis 95 % eines wasserfreien Emollients, welches bei Zimmertemperatur plastisch oder flüssig sein muss, sowie 5 bis 90 % eines sogenannten immobilizers, der einen Schmelzpunkt von mindestens 35 °C, aber vorzugsweise 40 °C aufweisen sollte.

Das Hauptproblem der bekannten Lotionen ist deren Lagerstabilität. Wesentlich ist, dass die Lotionen bei Hauttemperatur, also etwa 36 bis 38 °C so vorliegen, dass sie ohne Schwierigkeiten vom Vliesstoff auf die Haut übergehen können. Dieser temperaturgesteuerte Prozess kann aber dann zu Problemen führen, wenn die Hygieneprodukte bei höheren Temperaturen, beispielsweise mehr als 30 °C gelagert werden. Häufig beobachtet man dann, daß die Lotionen auf den Vliesstoffen "ausschwitzen". Es war daher die Aufgabe der vorliegenden Erfindung, hautfreundliche Lotionen zur Applikation auf Vliesstoffe für Hygieneartikel bereitzustellen, wobei deren Lagerstabilität insbesondere bei hohen Temperaturen gewährleistet sein muss.

Außerdem ist zu beachten, dass der Vliesstoff beispielsweise in Windeln, flüssigkeitsdurchlässig sein muss und daher üblicherweise hydrophil präpariert worden ist. Die weitere Ausrüstung mit einer in der Regel hydrophoben hautfreundlichen Lotion könnte daher den Flüssigkeitstransport durch das Vlies in die absorbierenden Materialien verringern oder deutlich verschlechtern.

Weiterhin ist es gewünscht, dass die Lotionen von dem Vliesstoff möglichst vollständig auf die Haut des Trägers übergehen und dabei ggf. weiteren Zusatznutzen vermitteln, beispielsweise eine Geruchsbildung oder aber das Wachsen von Bakterien, Pilzen und Hefen verringern können. Prinzipiell müssen die Lotionen natürlich leicht auf die Vliesstoffe applizierbar sein und sich möglichst mit den bekannten Präparationsverfahren anwenden lassen. Mit den wasserfreien Lotionen, die im Stand der Technik beschrieben sind, waren die oben aufgeführten Aufgaben nicht lösbar, insbesondere treten Schwierigkeiten bei der Applikation von bestimmten Additive auf, wie z.B. Zink-Ricinoleat oder Chitosanen, die erst wirksam werden, wenn sie aus wässerigem Medium appliziert werden.

Es wurde gefunden, daß durch Kombination ausgewählter Ölkörper geeignete wässerige Mittel erhalten werden, welche die obigen Anforderungen erfüllen.

Ein erste Gegenstand der Erfindung betrifft Mittel, enthaltend mindestens 5 bis 50 Gew.-% einer im Bereich von 25 bis 37°C schmelzende Komponente a), ausgewählt aus der Gruppe der Paraffine, Fettsäureester, Polyhydroxyfettsäureester, Fettalkohole, alkoxylierten Fettsäureester, alkoxylierten Fettalkohole und Mischungen dieser Verbindungen und 5 bis 50 Gew.-% einer im Bereich von 40 bis 60 °C schmelzende Komponente b), ausgewählt aus der Gruppe der Polyhydroxyfettsäureester, C₁₄-C₂₂-Fettalkohole, C₁₂-C₂₂-Fettsäuren, den alkoxylierten Derivaten der Fettalkohole und -ester, sowie Mischungen dieser Komponenten, und Glyceriden von Fettsäuren mit 8 bis 18 C-Atomen c) 5 bis 25 Gew.-% Wasser, und d) 1 bis 15 Gew.-% Polyglycerinpoly-12-hydroxystearat

Die erfindungsgemäßen Mittel stellen Emulsionen oder Suspensionen dar. Vorzugsweise liegen Emulsionen des O/W oder W/O-Typs vor.

Die Komponente a) kann ausgewählt werden aus einer Vielzahl dem Fachmann bekannten Verbindungen, wobei wesentlich ist, dass der Schmelzpunkt hier im Bereich von 25 bis max. 37 °C liegen muss. Zum einen können dazu bestimmte Paraffine aber auch Fettsäureester und insbesondere Fettalkohole eingesetzt werden. Bei den Paraffinen eignen sich vorzugsweise halbfeste Paraffine wie Weichparaffin, vorzugsweise Petrolatum. Geeignete Fettalkohole sind beispielsweise Dodecanol oder Ricinolalkohol, um einen Vertreter der ungesättigten Fettalkohole zu nennen. Besonders geeignet im Sinne der vorliegenden Erfindung ist der Einsatz von Glyceriden, hier vorzugsweise der Mischungen von Partial- und Triglyceriden, wobei diese den gewünschten Schmelzpunkt von 25 bis 37 °C aufweisen müssen. Besonders bevorzugt sind hier Mischungen von Glyceriden von Fettsäuren mit 8 bis 18 C-Atomen.

Glyceride stellen Mono- Di- und/oder Triester des Glycerins mit Fettsäuren, nämlich beispielsweise Capronsäure, Caprylsäure, 2--Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen dar. Sie folgen der Formel (I), 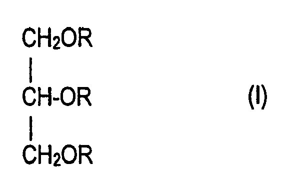 in der R für einen Rest COR', in dem R' einen verzweigten oder unverzweigten, gesättigten oder ungesättigten Alkylrest mit 6 bis 22 Kohlenstoffatomen bedeutet, und/oder unabhängig voneinander für Wasserstoff steht. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Isostearinsäuremonoglycerid, Isostearinsäurediglycerid, Ölsäuremonoglycerid, Ölsäurediglycerid, Talgfettsäuremonoglycerid, Talgfettsäurediglycerid, Behensäuremonoglycerid, Behensäurediglycerid, Erucasäuremonoglycerid, Erucasäurediglycerid sowie deren technische Gemische, die untergeordnet aus dem Herstellungsprozeß noch geringe Mengen an Triglycerid enthalten können.

Wesentlich für die vorliegende Erfindung ist der Einsatz der Emulgatorkomponente d). Hierbei handelt es sich um Polyglycerinpoly-12-hydroxystearat. Bei diesen handelt es sich um das Umsetzungsprodukt von Poly-12hydroxystearinsäure mit Polyglycerin. Dabei enthält das Polyglycerin die folgende Zusammensetzung: Glycerine 5 bis 35 Gew.-%, Diglycerine 15 bis 40 Gew.-%, Triglycerine 10 bis 35 Gew.-%, Tetraglycerine 5 bis 20 Gew.-%, Pentaglycerine 2 bis 10 Gew.-% und der Rest Oligoglycerine.

Neben den Komponente a) bis d) können die erfindungsgemäßen Mittel noch weitere Bestandteile enthalten, insbesondere weitere Emulgatoren, vorzugsweise nicht-ionische Emulgatoren. Nicht-ionische Emulgatoren zeichnen sich durch ihre Hautfreundlichkeit und Milde sowie ihre ökotoxologisch guten Eigenschaften aus. Durch Verwendung einer Kombination nicht-ionischer Emulgatoren erhält man besonders feinteilige Emulsionen, so daß die Stabilität der Zusammensetzung erhöht wird. Die erfindungsgemäß Zusammensetzung enthält die Co-Emulgatoren in einer Menge von 0 bis 15 Gew.-%, vorzugsweise1 bis 10 Gew.-% und insbesondere 3 bis 10 Gew.-% bezogen auf das Gesamtgewicht der Zusammensetzung.

Außerdem können in den erfindungsgemäßen Mitteln noch weitere übliche Inhaltsstoffe enthalten sein, beispielsweise Silikonwachse bzw. Polysiloxane in Mengen von 1 bis 6 Gew.-%, vorzugsweise 1,5 bis 5,5 Gew.-% und insbesondere von 2 bis 5 Gew.-%. Polysiloxane sind bekannte polymere Verbindungen, die als Monomereinheiten die folgende Struktur enthalten:

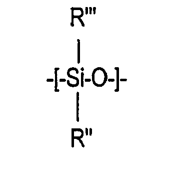

Dabei steht R" bzw. R'" unabhängig voneinander für Wasserstoff, oder eine Alkyl-, Cycloalkyl-, Aryl- oder Alkenylrest. Vorzugsweise weisen derartige Siloxane Viskositäten bei 37 °C im Bereich von 5 bis 5000 mPA s auf.

Weiterhin und bevorzugt können die erfindungsgemäßen Mittel hautfreundliche bzw. hautpflegende Substanzen, vorzugsweise in Mengen von 0,1 bis 10 Gew.-%, insbesondere 1 bis 8 Gew.-% und ganz besonders bevorzugt von 2 bis 6 Gew.-% enthalten. Derartige Inhaltsstoffe können beispielsweise Bisabolol, Alantoin und Panthenol sein. Auch Vitamine, vorzugsweise Vitamin E und Vitaminvorstufen sowie Proteinhydrolysate können eingesetzt werden. Geeignet sind weiterhin Pflanzenextrakte, vorzugsweise aus Kamille, Aloe Vera, Lindenblüten, Rosskastanien, grünem Tee, Eichenrinde, Brennnessel, Hopfen, Klettenwurzel, Schachtelhalm, Weißdom, Mandel, Fichtennadel, Mandelholz, Wacholder, Kokosnuss, Aprikose, Limone, Weizen, Kiwi, Melone, Orange, Grapefruit, Salbei, Rosmarin, Birke, Malve, Scharfgarbe, Thymian, Melisse, Hau, Hechel, Huflattich, Eibisch, Ginseng und Ingwerwurzeln. Daneben können aber noch andere hautpflegende Substanzen enthalten sein. Hier sei insbesondere namentlich benannt das Chitosan sowie Zinkoxid oder Zink-Ricinoleat.

In einer besonderen Ausführungsform der Erfindung können die Emulsionen weitere Hilfs- und Zusatzstoffe, wie beispielsweise Überfettungsmittel, Verdickungsmittel, Polymere, Wachse, biogene Wirkstoffe, Deowirkstoffe, Filmbildner, UV-Lichtschutzfaktoren, Antioxidantien, Hydrotrope, Konservierungsmittel, Insektenrepellentien, Selbstbräuner, Solubilisatoren, Stabilisatoren, Parfümöle, Farbstoffe, keimhemmende Mittel und dergleichen enthalten.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole^{®} von Goodrich oder Synthalene^{®} von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte VinylpyrrolidonNinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, α-Hydroxycarbonsäuren, Aminosäuren, Ceramide, Pseudoceramide, essentielle Öle, Pflanzenextrakte und Vitaminkomplexe zu verstehen.

Als Deowirkstoffe kommen z. B. Antitranspirantien wie etwa Aluminiumchlorhydrate, Aluminium-Zirkonium-Chlorohydrate sowie Zinksalze in Frage. Diese werden zur Herstellung von schweißhemmenden und desodorierenden Zubereitungen eingesetzt und wirken wahrscheinlich über den partiellen Verschluß der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden. Unter der Marke Locron^{®} der Clariant GmbH, befindet beispielsweise sich ein Aluminiumchlorhydrat im Handel, das der Formel [Al₂(OH)₅Cl]·2,5 H₂O entspricht und dessen Einsatz besonders bevorzugt ist. Ebenso erfindungsgemäß bevorzugt ist der Einsatz von Aluminium-Zirkonium-Tetrachlorohydrex-Glycin-Komplexen, die beispielsweise von Reheis unter der Bezeichnung Rezal^{®} 36G vermarktet werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} C.A.T., Cognis Deutschland GmbH). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Emulsionen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan^{®} von der Ciba-Geigy, Basel/CH vertrieben wird.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Der Gesamtanteil der Hilfs- und Zusatzstoffe kann 1 bis 50, vorzugsweise 5 bis 40 Gew.-% - bezogen auf die Mittel - betragen.

Weiterhin kann es vorteilhaft sein Hilfsmittel zur Stabilisierung der Emulsion mitzuverwenden, beispielsweise Glycerin oder Magnesiumsulfat, vorzugsweise in Mengen von jeweils 0,1 bis maximal 5 Gew.-%, vorzugsweise von 0,1 bis 1,5 Gew.-%.

Die Herstellung der Mittel kann durch übliche Kalt - oder Heißprozesse erfolgen; vorzugsweise arbeitet man nach der Phaseninversionstemperatur-Methode.

Ein weiterer Gegenstand der vorliegenden Erfindung betrifft die Verwendung von Mittel gemäß obiger Beschreibung zur hautfreundlichen Ausrüstung von Vliesstoffen.

Bei den Vliesstoffen handelt es sich um dem Fachmann bekannte Substanzen. Als Vliesstoffe werde im Rahmen der vorliegenden Erfindung vorzugsweise solche eingesetzt, die vollständig oder teilweise Polyolefine enthalten. Hier eigenen sich an sich alle heute bekannten Polymer- und Copolymertypen auf Ethylen- beziehungsweise Propylen-Basis. Auch Abmischungen reiner Polyolefine mit Copolymeren sind grundsätzlich geeignet. Für die erfindungsgemäße Lehre besonders geeignete Polymertypen sind in der nachfolgenden Zusammenstellung aufgezählt: Poly(ethylene) wie HDPE (high density polyethylene), LDPE (low density polyethylene), VLDPE (very low density polyethylene), LLDPE (linear low density polyethylene), MDPE (medium density polyethylene), UHMPE (ultra high molecular polyethylene), VPE (vernetztes Polyethylen), HPPE (high pressure polyethylene); isotaktisches Polypropylen; syndiotaktisches Polypropylen; Metallocen-katalysiert hergestelltes Polypropylen, schlagzähmodifiziertes Polypropylen, Random-Copolymere auf Basis Ethylen und Propylen, Blockcopolymere auf Basis Ethylen und Propylen; EPM (Poly[ethylen-co-propylen]); EPDM (Poly[ethylen-co-propylen-co-konjugiertes Dien]). Weitere geeignete Polymertypen sind: Poly(styrol); Poly(methylstyrol); Po-ly(oxymethylen); Metallocen-katalysierte alpha-Olefin- oder Cycloolefin-Copolymere wie Norbornen-Ethylen-Copolymere; Copolymere, die zu mindestens 80 % Ethylen und/oder Styrol enthalten und zu weniger als 20 % Monomere wie Vinylacetat, Acrylsäureester, Methacrylsäureester, Acrylsäure, Acrylnitril, Vinylchlorid. Beispiele solcher Polymeren sind: Poly(ethylen-co-ethylacrylat), Poly(ethylen-co-vinylacetat), Poly(ethylen-co-vinylchlorid), Poly(styrol-co-acrylnitril). Geeignet sind weiterhin Pfropfcopolymere sowie Polymerblends, das heißt, Mischungen von Polymeren, in denen unter anderem die vor-genannten Polymere enthalten sind, beispielsweise Polymerblends auf Basis von Polyethylen und Polypropylen.

Im Rahmen der vorliegenden Erfindung sind Homo- und Copolymere auf Basis von Ethylen und Propylen besonders bevorzugt. In einer Ausführungsform der vorliegenden Erfindung setzt man dementsprechen als Polyolefin ausschließlich Polyethylen ein, in einer anderen Ausführungsform ausschließlich Polypropylen, in einer weiteren Ausführungsform Copolymere auf Basis von Ethylen und Propylen.

Ein weiterer Gegenstand der Erfindung ist die Verwendung der gemäß dem oben beschriebenen Verfahren hergestellten hydrophilisierten und durch wäßrige Medien benetzbaren Fasern auf Polyolefin bzw. Polyester-Basis zur Herstellung textiler Flächengebilde. Vorzugsweise sind dabei die textilen Flächengebilde Vliesstoffe. In einer besonders bevorzugten Ausführungsform sind diese textilen Flächengebilde zum Einsatz in Windeln bestimmt.

### Beispiele

Es wurden die folgenden erfindungsgemäßen Mittel hergestellt:

### Emulsion 1

48 Gew.-% Partial-/Triglyceridmischung auf Basis C₁₄/C₁₆-Fettsäuren, Schmelzbereich: 33,0-35,5 °C
15 Gew.-% Partial-/Trigylceridmischung auf Basis C14/C6-Fettsäuren, Schmelzbereich 40,0-42,0 °C
10 Gew.-% Polyhydroxystearinsäurepolyglycerinester
5 Gew.-% ZnO
1,45 Gew.-% Glycerin (86 %ig)
Rest Wasser

Die Emulsion wurde mittels Slotcoating mit einem Gerät der Fa. Nordson auf PP-Vliese in einer Konzentration von 10 und 30 mg/l appliziert.

## Patentansprüche

1. Mittel, enthaltend mindestens
a) 5 bis 50 Gew.-% einer im Bereich von 25 bis 37 °C schmelzende Komponente, ausgewählt aus der Gruppe der Paraffine, Fettsäureester, Polyhydroxyfettsäureester, Fettalkohole, alkoxylierten Fettsäureester, alkoxylierten Fettalkohole und Mischungen dieser Verbindungen und
b) 5 bis 50 Gew.-% einer im Bereich von 40 bis 60 °C schmelzende Komponente, ausgewählt aus der Gruppe der Polyhydroxyfettsäureester, C₁₄-C₂₃-Fettalkohole, C₁₂-C₂₂-Fettsäuren, den alkoxylierten Derivaten der Fettalkohole und -ester sowie Mischungen dieser komponenten, und Glyceriden von Fettsäuren mit 8 bis 18 C-Atomen,
c) 5 bis 25 Gew.-% Wasser und
d) 5-15 Gew.-% Polyglycerinpoly-12-hydroxystearat.

2. Mittel nach Anspruch 1, **dadurch gekennzeichnet, dass** es als W/O oder O/W-Emulsion vorliegt.

3. Mittel nach den Anspruch 1 oder 2, **dadurch gekennzeichnet, dass** die wässerigen Mittel eine Viskosität bei 23 °C von 100 bis 100000 mPa s aufweisen.

4. Verwendung von Mitteln gemäß den Ansprüchen 1 bis 3 zur hautfreundlichen Ausrüstung von Vliesstoffen.

## Claims

1. A composition containing at least
a) 5 to 50% by weight of a component melting at 25 to 37°C selected from the group of paraffins, fatty acid esters, polyhydroxyfatty acid esters, fatty alcohols, alkoxylated fatty acid esters, alkoxylated fatty alcohols and mixtures of these compounds,
b) 5 to 50% by weight of a component melting at 40 to 60°C selected from the group of polyhydroxyfatty acid esters, C₁₄₋₂₂ fatty alcohols, C₁₂₋₂₂ fatty acids, alkoxylated derivatives of the fatty alcohols and esters and mixtures of these components and glycerides of C₈₋₁₈ fatty acids,
c) 5 to 25% by weight water and
d) 5 to 15% by weight polyglycerol poly-12-hydroxystearate.

2. A composition as claimed in claim 1, **characterized in that** it is formulated as a w/o or o/w emulsion.

3. A composition as claimed in claim 1 or 2, **characterized in that** the water-based compositions have a viscosity at 23°C of 100 to 100,000 mPa.s.

4. The use of the compositions claimed in claims 1 to 3 for the dermatologically compatible finishing of nonwovens.

## Revendications

1. Agent contenant au moins :
a) 5 à 50 % en poids d'un composant dont la fusion se produit dans la plage de 25 à 37 °C, choisi dans le groupe des paraffines, des esters d'acides gras, des esters d'acides gras polyhydroxylés, des alcools gras, des esters d'acides gras alcoxylés, des alcools gras alcoxylés et des mélanges de ces composés et
b) 5 à 50 % en poids d'un composant dont la fusion se produit dans la plage de 40 à 60 °C, choisi dans le groupe des esters d'acides gras polyhydroxylés, des alcools gras en C₁₄-C₂₂, des acides gras en C₁₂-C₂₂, des dérivés alcoxylés des alcools gras et des esters gras ainsi que des mélanges de ces composants, et des glycérides d'acides gras ayant 8 à 18 atomes de carbone,
c) 5 à 25 % en poids d'eau et
d) 5 à 15 % en poids de poly-12-hydroxystéarate de polyglycérol.

2. Agent selon la revendication 1,
**caractérisé en ce qu'**
il se présente sous la forme d'une émulsion E/H ou H/E.

3. Agent selon la revendication 1 ou 2,
**caractérisé en ce que**
les agents aqueux présentent une viscosité à 23 °C de 100 à 100 000 mPa•s.

4. Utilisation d'agents selon les revendications 1 à 3, pour le finissage adoucissant pour la peau, d'étoffes nappées.
